# EUROPEAN PATENT SPECIFICATION

(11) **EP 0 414 049 B1**
(45) Date of publication and mention of the grant of the patent: **05.05.1993**
(21) Application number: 90115223.1
(22) Date of filing: 08.08.1990
(51) Int. Cl.: C09C 1/00, C09C 1/28, C09C 1/42, C09C 1/40

(54) **Pigments having an effect of preventing ultraviolet rays and infrared rays**
Pigmente, welche die ultraviolette und infrarote Strahlen zurückhalten
Pigments retenant les rayons ultraviolets et infrarouges

(30) Priority: 21.08.1989 JP 212972/89
(43) Date of publication of application: 27.02.1991
(73) Proprietor: MERCK PATENT GmbH, D-64271 Darmstadt (DE)
(72) Inventor: Noguchi, Tamio, Iwaki-shi, Fukushima-ken (JP); Aikawa, Masahiro, 1-chome, Iwaki-shi, Fukushima-ken (JP)

(56) References cited:
- EP-A- 0 096 284
- EP-A- 0 142 695
- EP-A- 0 268 918
- DE-A- 2 313 331

## Description

### Detailed Description of the Invention

### (Field of Industrial Application)

The present invention relates to novel pigments which possess good adhesion and spreadability and have an effect of preventing ultraviolet rays and infrared rays that cause erythematous response or pigmentation..

### (Prior Art)

It has been pointed out that, when the skin is exposed to ultraviolet rays or infrared rays in the sunlight, skin temperature increases due to infrared rays, whereby the photochemical reaction of ultraviolet rays is accelerated so that so-called sunburn tends to be caused. Furthermore, near infrared rays at 760 nm to 1,500 nm could be a factor to cause low temperature burn or skin aging change accompanied by evaporation of epidermal moisture, so-called photoaging, etc. As cosmetics for protecting light, there have been recently provided cosmetic compositions in which UV absorbents of organic compound type such as quinine sulfate or p-aminobenzoic acid are incorporated or to which an effect of shielding ultraviolet rays at 300 to 400 nm is added by utilizing a light scattering effect of inorganic powders, for example, zinc oxide or titanium oxide.

In addition, as powders for scatterring ultraviolet rays and infrared rays at the same time, nylon particles having bound zirconium oxide thereto or aluminum powders treated with titanium oxide have been developed. However, these prior art cosmetics do not have good adhesion or spreadability to the skin and are also unsatisfactory in other desired properties such as smoothness, soft touch, transparency, color hue, etc. and in the effect of preventing ultraviolet rays and infrared rays.

Furthermore, organic compounds that prevent ultraviolet rays and infrared rays at the same time are unknown, have irritation to the skin and are insufficient in stability to light. In particular, a sufficient effect of reflecting near infrared rays at 760 nm to 1,500 nm is not recognized in the organic compounds.

West German Patent No. DE-A-3221045 discloses a titanium oxide-covered mica type pearlescent pigment; in preparing the pigment, a trace amount of zirconium oxide is deposited together with titanium oxide for purposes of improving its luminance and adhesion of titanium oxide. However, the pigment has no sufficient infrared ray reflection property. Furthermore, the luminance is so strong that is unsuited as powder foundation, etc. for use.

In addition, the infrared ray reflection property of pearlescent pigment having a red-interference color which is obtained by covering mica with 50 to 60 wt% of titanium oxide is reported to Poelman et al. [M.C. Poelman, et al., Preprints of the XIVth I.F.S. Congress Barcelona, vol. II, 749 (1986)].

However, in this pigment the luminance is also strong so that it is undesired for use of powder foundation.

### (Disclosure of the Invention)

As a result of extensive investigations to find pigments having good adhesion and spreadability to the skin, having smoothness, soft touch, transparency and gloss and hue to a suitable extent and having an effect of preventing ultraviolet rays and infrared rays, the present inventors have succeeded in providing novel pigments which are obtained by covering a platelet-like substrate having a particle diameter of 0.5 to 100 µm with a metal oxide mixture of titanium oxide and zirconium oxide in a suitable ratio.

That is, the present invention provides novel pigments having an effect of preventing ultraviolet rays and infrared rays comprising platelet-like substrate particles such as mica, talc, sericite, kaolin. having a particle diameter of 0.5 to 100 µm characterized by covering the surface of the particles with a metal oxide comprising titanium oxide and zirconium oxide in a molar ratio of 10:1 to 10:15 within a range of from 20 wt% to 60 wt% based on the total weight of the pigment. The present invention also relates to novel pigments obtained by previously covering the surface of platelet-like substrate particles with barium sulfate and further covering with the aforesaid metal oxide composed of titanium oxide and zirconium oxide, and a process for preparation thereof.

The novel pigments in accordance with the present invention can be prepared as described below, the process conditions being specified below pages 7-9 and in claim 3.
(1) Firstly, the aforesaid platelet-like substrate particles are suspended in water.
(2) Next, (a) an aqueous solution of a titanium compound and a zirconium compound and (b) an aqueous solution containing an alkaline hydroxide or an alkaline carbonate are added to the suspension, simultaneously or sequentially in the order of (a) and (b), with heating while stirring, thereby to deposit the hydrated titanium oxide and hydrated zirconium oxide onto the surface of the platelet-like substrate particles.
(3) The resulting solid product is separated, dried and if necessary, sintered.

The thus obtained platelet-like substrate particles on which titanium oxide and zirconium oxide have been deposited are extremely excellent in reflection properties of ultraviolet rays and infrared rays.

For purposes of improving spreadability and adhesion to the skin and gloss (luster, brightness) of powders, the present invention also provides novel pigments comprising platelet-like substrate particles obtained by previously covering the surface of the particles with barium sulfate and further covering the surface of the particles with the aforesaid metal oxide composed of titanium oxide and zirconium oxide. In this case, the pigment can be prepared as described below, the press conditions being openified on pages 7-9, and in claim 4.
(1) The platelet-like substrate particles are suspended in water.
(2) Next, (c) an aqueous solution containing barium ions (a) an aqueous solution containing titanium sulfate and a zirconium compound in water to the suspension, simultaneously or sequentially in the order of (c) and (a), with heating while stirring, thereby to previously deposit barium sulfate onto the surface of the platelet-like substrate particles and, further adding (b) an aqueous solution containing an alkaline hydroxide or an alkaline carbonate to the mixture, with heating while stirring, thereby to deposit barium sulfate, hydrated titanium oxide and hydrated zirconium oxide onto the surface of the platelet-like substrate particles.
(3) The resulting solid product is separated, dried and if necessary, sintered.

The thus obtained platelet-like substrate particles onto the surface of which barium sulfate, hydrated titanium oxide and hydrated zirconium oxide have been deposited have good adhesion and spreadability to the skin, have smoothness, soft touch, transparency and gloss and hue to a suitable extent and are excellent in the effect of preventing ultraviolet rays and infrared rays.

Examples of the titanium compound used in the aqueous solution (a) described above include titanium tetrachloride, titanium trichloride, titanyl oxysulfate. Examples of the zirconium compound include zirconium chloride oxide octahydrate, zirconium sulfate, etc. Examples of the alkaline oxide in the aqueous solution (b) described above include sodium hydroxide, potassium hydroxide, and ammonium hydroxide. In this case, compounds that form ammonium hydroxide, for example, urea and the like may also be used.

Examples of the alkaline carbonate described above include carbonates of alkali metals such as sodium carbonate, sodium hydrogencarbonate, potassium hydrogencarbonate, ammonium carbonate, ammonium hydrogencarbonate.

Examples of the aqueous solution containing barium ions in the aqueous solution (c) described above include aqueous solutions of barium chloride, barium hydroxide, barium nitrate, barium sulfide.

Of these barium compounds, barium nitrate is a preferred compound in view of its accessibility, price, purity, etc.

The particle diameter of the platelet-like substrate particles used in the present invention is 0.5 to 100 µm, preferably 2 to 50 µm. As the platelet-like substrate, clay minerals such as mica, talc, sericite, kaolin. are used.

The process for preparing the novel pigments of the present invention described above is described below.

In 100 parts of water is suspended 5 parts of platelet-like substrate particles. While stirring at 0 to 100°C, preferably 20 to 95°C, an aqueous solution containing 5 to 35 wt% of the titanium compound is added to an aqueous solution containing 5 to 35 wt% of the zirconium compound which contains 0.1 to 1.5 molar equivalents based on the titanium compound. The resulting solution (the aqueous solution (a) described above) is added to 5 to 35 wt% of a basic aqueous solution (the aqueous solution (b) described above) with stirring, while controlling the pH during the reaction to 1.5 to 2.5. The pH after the reaction is adjusted to the pH that the metal hydroxide is completely deposited.

After the reaction, the solid product is separated by filtration and washed with water. Then, the product is dried at 105 to 110°C for about 12 hours. In the case of converting the metal hydroxide to the oxide, the resulting solid product is sintered at a suitable temperature.

Next, for purposes of improving spreadability and adhesion to the skin and gloss (luster, brightness) of powders, the surface of the platelet-like substrate particles is previously covered with barium sulfate and the metal oxide composed of titanium oxide and zirconium oxide is coated onto the particle surface. This process is described below.

After 5 parts of the platelet-like substrate particles are suspended in 100 parts of water, an aqueous solution containing 5 to 25 wt% of a barium salt, 1.01 to 2.00 molar equivalents of titanyl sulfate to the barium salt and 0.1 to 1.5 molar equivalents of the zirconium compound to the titanyl sulfate is dropwise added to the suspension at 0 to 100°C, preferably 20 to 95°C, while stirring. After completion of the dropwise addition, 5 to 35 wt% of a basic aqueous solution (the aqueous solution (b) described above) is dropwise added to the mixture and a pH in the aqueous solution is adjusted to 3.0 to 10.0.

After the reaction, the solid product is separated by filtration and washed with water. Then, the product is dried at 105 to 110°C for about 12 hours. In the case of converting the metal hydroxide to the oxide, the resulting solid product is sintered at a suitable temperature.

Hereafter the present invention is described in detail by referring to the examples but is not deemed to be limited to these examples.

### Example 1

After 50 g of talc particles having a particle diameter of 1 to 30 microns was suspended in 1,000 ml of water, 50 ml of ethanol was added to the suspension to improve dispersibility of the talc particles in water. While stirring with heating at 70°C, a solution obtained by adding 55 ml of an aqueous solution containing 15 wt% of zirconium chloride oxide octahydrate to 300 ml of an aqueous solution containing 15 wt% of titanium tetrachloride was dropwise added to the mixture at a rate of 3 ml/min.

The pH of the suspension was adjusted to 1.5 to 2.5 by adding an aqueous solution containing 15 wt% of sodium hydroxide thereto. After completion of the dropwise addition, the pH of the suspension was adjusted to 6.5 using an aqueous solution containing 15 wt% of sodium hydroxide.

The resulting product was separated by filtration and washed with water to remove the salt. Then, the product was dried at 105 to 110°C for 13 hours and sintered at 700°C for an hour.

The thus obtained pigment showed good spreadability and adhesion to the skin, excellent dispersibility and excellent reflection effect to ultraviolet rays and infrared rays.

### Example 2

After 50 g of kaolin having a diameter of 20 microns or less was suspended in 1,000 ml of water, a solution obtained by adding 200 ml of an aqueous solution containing 15 wt% of zirconium chloride oxide octahydrate to 300 ml of an aqueous solution containing 15 wt% of titanium tetrachloride was dropwise added to the suspension at a rate of 3 ml/min.

The pH of the suspension was adjusted to 1.5 to 2.5 by adding an aqueous solution containing 15 wt% of sodium hydroxide thereto. After completion of the dropwise addition, the pH of the suspension was adjusted to 6.5 using an aqueous solution containing 15 wt% of sodium hydroxide.

The resulting product was separated by filtration and washed with water to remove the salt. Then, the product was dried at 105 to 110°C for 13 hours and sintered at 700°C for an hour.

The thus obtained white product reflected ultraviolet rays and infrared rays.

### Example 3

After 70 g of silk mica particles having a particle diameter of 1 to 40 microns was suspended in 1 liter of water, the mixture was heated to 85°C. While stirring, a solution mixture of 200 g of an aqueous solution containing 34 wt% of titanyl sulfate and 80 g of an aqueous solution containing 30 wt% of zirconium chloride oxide octahydrate was dropwise added to the mixture at a rate of 2 ml/min. After completion of the dropwise addition, the pH of the suspension was adjusted to 7.0 while dropwise adding an aqueous solution containing 30 wt% of sodium hydroxide at a rate of 1.5 ml/min to the mixture.

The resulting product was separated by filtration and washed with water to remove the salt. Then, the product was dried at about 105 to 110°C for 15 hours and sintered at 700°C for 40 minutes.

The thus obtained white product showed excellent reflection effect to ultraviolet rays and infrared rays.

### Example 4

To an aqueous solution of 100.8 g of barium nitrate in 2 liters of water was added 90 mg of white mica particles having a particle diameter of 1 to 15 microns to suspend the particles. The suspension was heated to 80°C and a solution mixture of 530 g of an aqueous solution containing 34 wt% of titanyl sulfate and 261.7 g of an aqueous solution containing 30 wt% of zirconium chloride oxide octahydrate was dropwise added to the suspension at a rate of 5.4 ml/min. After completion of the dropwise addition, the pH of the suspension was adjusted to 7.0 while dropwise adding an aqueous solution containing 30 wt% of sodium hydroxide at a rate of 3.0 ml/min to the mixture. The resulting product was precipitated, filtered and washed with water to remove the salt. Then, the product was dried at 105 to 110°C for 15 hours and sintered at 700°C for 40 minutes. The thus obtained product showed extremely excellent reflection effect to ultraviolet rays and infrared rays.

### Example 5

The procedure was operated in a manner similar to Example 4 except that the amount of barium nitrate, the heating temperature, the amount of the aqueous solution containing 34 wt% of titanyl sulfate used and the amount of the aqueous solution containing 30 wt% of zirconium chloride oxide octahydrate were changed to 84 g, 90°C, 441.7 g and 523 g, respectively, in Example 4. The obtained product showed excellent reflection effect to ultraviolet rays and infrared rays.

### Example 6

The procedure was operated in a manner similar to Example 5 except that the amount of the aqueous solution containing 34 wt% of titanyl sulfate used and the amount of the aqueous solution containing 30 wt% of zirconium chloride oxide octahydrate were changed to 353.4 g and 653.8 g, respectively, in Example 5. The obtained white product showed extremely excellent reflection effect to ultraviolet rays and infrared rays.

### Example 7

The procedure was operated in a manner similar to Example 4 except that silk mica particles having a particle diameter of 1 to 40 microns were used instead of white mica particles. The white powders showing excellent reflection effect to ultraviolet rays and infrared rays were obtained.

The pigment obtained in each of the examples described above was dispersed in a medium of polyvinyl chloride type having a solid content of 20%. The dispersion was coated onto a glass plate in a wet state using an applicator having a thickness of 120 µm. After drying, a membrane was obtained. Using the membrane, the ultraviolet region (400 to 260 nm) and the near infrared region (760 to 900 nm) were measured with a double beam spectrophotometer Model 228 (manufactured by Hitachi Ltd.). The light transmission at wavelengths of 300 nm and 900 nm was less than 10% and less than 30%, respectively.

## Claims

1. A pigment having an effect of preventing ultraviolet rays and infrared rays comprising platelet-like substrate particles having a particle diameter of 0.5 to 100 µm characterized in that the surface of said particles is covered with a metal oxide comprising titanium oxide and zirconium oxide in a molar ratio of 10:1 to 10:15 within a range of from 20 wt% to 60 wt% based on the total weight of the pigment.

2. A pigment as claimed in claim 1, wherein the surface of said platelet-like substrate particles is previously covered with barium sulfate.

3. A process for preparing a pigment having an effect of preventing ultraviolet rays and infrared rays which comprises suspending platelet-like substrate particles having a particle size of 0.5 to 100 µm in water; adding at 0 to 100 °C (a) an aqueous solution of a titanium compound and a zirconium compound and (b) 5 to 35 wt% of an aqueous solution of an alkaline hydroxide or an alkaline carbonate to the suspension, (a) and (b) simultaneously or (a) and then (b), with stirring, while controlling the pH during the reaction to 1.5 to 2.5, thereby to deposit titanium hydroxide and zirconium hydroxide onto the surface of the platelet-like substrate particles; separating the resulting solid product, drying and sintering characterized in that the surface of said particles is covered with a metal oxide comprising titanium oxide and zirconium oxide in a molar ratio of 10:1 to 10:15 within a range of from 20 wt% to 60 wt% based on the total weight of the pigment.

4. A process for preparing a pigment having an effect of preventing ultraviolet rays and infrared rays which comprises suspending platelet-like substrate particles having a particle size of 0.5 to 100 µm in water; adding (c) an aqueous solution containing 5 to 25 wt% of a barium salt and (d) an aqueous solution of a titanium sulfate and a zirconium compound in water to the suspension at 0 to 100 °C, (c) and (d) simultaneously or (c) and then (d), with stirring, thereby to deposit barium sulfate onto the surface of the leaf-like substrate particles; further adding (b) 5 to 35 wt% of an aqueous solution of an alkaline hydroxide or an alkaline carbonate to the mixture while controlling the pH during the reaction to 3.0 to 10.0 to deposit titanium hydroxide and zirconium hydroxide onto the surface of the platelet-like substrate particles; separating the resulting solid product, drying and sintering.

## Patentansprüche

1. Gegen Ultraviolett- und Infrarotstrahlen schützendes Pigment, bestehend aus plättchenförmigen Substratteilchen mit einem Teilchendurchmesser von 0,5 bis 100 µm, dadurch gekennzeichnet, daß die Oberfläche der Teilchen mit einem aus Titanoxid und Zirkonoxid in einem Molverhältnis von 10 zu 1 bis 10 zu 15 bestehenden Metalloxid in einem Mengenverhältnis von 20 bis 60 Gew.-%, bezogen auf das Gesamtgewicht des Pigments, belegt ist.

2. Pigment nach Anspruch 1, dadurch gekennzeichnet, daß die Oberfläche der plättchenförmigen Substratteilchen mit Bariumsulfat vorbelegt war.

3. Verfahren zur Herstellung eines gegen Ultraviolett- und Infrarotstrahlen schützenden Pigments, bei dem man plättchenförmige Substratteilchen einer Teilchengröße von 0,5 bis 100 µm in Wasser suspendiert, bei 0 bis 100°C (a) eine wäßrige Lösung einer Titanverbindung und einer Zirkonverbindung sowie (b) 5 bis 35 Gew.-% einer wäßrigen Lösung eines alkalischen Hydroxids oder alkalischen Carbonats gleichzeitig oder in der Reihenfolge (a) und dann (b) unter Rühren und bei Einhaltung eines pH-Werts von 1,5 bis 2,5 zugibt, um Titanhydroxid und Zirkonhydroxid auf der Oberfläche der plättchenförmigen Substratteilchen abzuscheiden, und das dabei entstehende Festprodukt abtrennt, trocknet und sintert, dadurch gekennzeichnet, daß man die Oberfläche der Teilchen mit einem aus Titanoxid und Zirkonoxid in einem Molverhältnis von 10 zu 1 bis 10 zu 15 bestehenden Metalloxid in einem Mengenverhältnis von 20 bis 60 Gew.-%, bezogen auf das Gesamtgewicht des Pigments, belegt.

4. Verfahren zur Herstellung eines gegen Ultraviolett- und Infrarotstrahlen schützenden Pigments, dadurch gekennzeichnet, daß man plättchenförmige Substratteilchen einer Teilchengröße von 0,5 bis 100 µm in Wasser suspendiert, bei 0 bis 100°C (c) eine wäßrige Lösung enthaltend 5 bis 25 Gew.-% eines Bariumsalzes und (d) eine wäßrige Lösung eines Titansulfats und einer Zirkonverbindung in Wasser gleichzeitig oder nacheinander in der Reihenfolge (c) und dann (d) unter Rühren zugibt, um Bariumsulfat auf der Oberfläche der plättchenförmigen Substratteilchen abscheidet, weiterhin (b) 5 bis 35 Gew.-% einer wäßrigen Lösung eines alkalischen Hydroxids oder eines alkalischen Carbonats unter Einhaltung eines pH-Werts von 3,0 bis 10,0 zugibt, um Titanhydroxid und Zirkonhydroxid auf der Oberfläche der plättchenförmigen Substratteilchen abzuscheiden, und das dabei entstehende Festprodukt abtrennt, trocknet und sintert.

## Revendications

1. Pigment capable de retenir les rayons ultraviolets et les rayons infrarouges, comprenant des particules en forme de plaquettes d'un substrat, le diamètre des particules étant compris entre 0,5 et 100 µm, caractérisé en ce que la surface desdites particules est enrobée d'un oxyde métallique comprenant de l'oxyde de titane et de l'oxyde de zirconium en un apport molaire de 10:1 à 10:15 dans la gamme de 20% à 60% en poids par rapport au poids total du pigment.

2. Pigment selon la revendication 1, dans lequel la surface desdites particules de substrat en forme de plaquettes est enrobée au préalable de sulfate de baryum.

3. Procédé de préparation d'un pigment ayant la capacité de retenir les rayons ultraviolets et les rayons infrarouges, qui consiste à mettre en suspension des particules de substrat analogues à des plaquettes ayant une granulométrie de 0,5 à 100 µm dans l'eau ; à ajouter à une température de 0 à 100°C (a) une solution aqueuse d'un composé de titane et d'un composé de zirconium et (b) de 5 à 35% en poids d'une solution aqueuse d'un hydroxyde alcalin ou d'un carbonate alcalin à la suspension, soit simultanément (a) et (b), soit d'abord (a) et, ensuite, (b), sous agitation, tout en réglant le pH en cours de réaction entre 1,5 et 2,5, pour déposer ainsi de l'hydroxyde de titane et de l'hydroxyde de zirconium sur la surface des particules de substrat analogues à des plaquettes; à séparer le produit solide résultant, à le sécher et à fritter, caractérisé en ce que la surface desdites particules est recouverte d'un oxyde métallique comprenant de l'oxyde de titane et de l'oxyde de zirconium dans un rapport molaire de 10:1 à 10:15 en dedant d'une gamme de 20 à 60% en poids par rapport au poids total du pigment.

4. Procédé de préparation d'un pigment ayant un effet de rétention des rayons ultraviolets et des rayons infrarouges, qui consiste à mettre en suspension des particules de substrat analogues à des plaquettes ayant une grosseur de 0,5 à 100 µm dans l'eau ; à ajouter (c) une solution aqueuse contenant de 5 à 25% en poids d'un sel de baryum et (d) une solution aqueuse d'un sulfate de titane et d'un composé de zirconium dans l'eau à la suspension à une température de 0 à 100°C, soit les composés (c) et (d) simultanément, soit d'abord (c) et, ensuite, (d), avec agitation pour ainsi déposer du sulfate de baryum à la surface des particules de substrat analogues à des feuilles, puis à ajouter (b) de 5 à 35% en poids d'une solution aqueuse d'un hydroxyde alcalin ou d'un carbonate alcalin au mélange tout en réglant le pH en cours de réaction à une valeur de 3,0 à 10,0 afin de déposer de l'hydroxyde de titane et de l'hydroxyde de zirconium sur les surfaces des particules en forme de plaquettes de substrat ; à séparer le produit solide résultant, à le sécher et à le fritter.
